# FASCICULE DE BREVET EUROPEEN

(11) **EP 0 800 399 B1**
(45) Date de publication et mention de la délivrance du brevet: **14.08.2002**
(21) Numéro de dépôt: 96901387.9
(22) Date de dépôt: 12.01.1996
(51) Int. Cl.: A61K 31/70, A61K 33/24, A61K 31/7105, A61K 31/711, A61P 35/00

(54) **TRAITEMENT THERAPEUTIQUE COMBINE DES PATHOLOGIES HYPERPROLIFERATIVES**
KOMBINIERTES THERAPEUTISCHES VERFAHREN ZUR BEHANDLUNG VON HYPERPROLIFERATIVEN KRANKHEITEN
COMBINED THERAPEUTICAL TREATMENT OF HYPERPROLIFERATIVE DISEASES

(30) Priorité: 17.01.1995 FR 9500436
(43) Date de publication de la demande: 15.10.1997
(73) Titulaire: Aventis Pharma S.A., 92165 Antony Cédex (FR)
(72) Inventeur: TOCQUE, Bruno, 92400 Courbevoie (FR)
(74) Mandataire: Savina, Jacques
(86) Numéro de dépôt international: FR9600056
(87) Numéro de publication internationale: WO9622101

(56) Documents cités:
- WO-A-87/05943

## Description

La présente invention concerne le domaine de la thérapie des pathologies hyperprolifératives. Elle concerne plus particulièrement un nouveau mode de traitement des pathologies hyperprolifératives basé sur l'utilisation combinée de deux types d'agents thérapeutiques.
Plus spécifiquement, la présente invention concerne un nouveau mode de traitement des pathologies hyperprolifératives basé sur l'utilisation combinée de gènes bloquant des voies de signalisation cellulaires oncogéniques et d'agents de chimiothérapie et ou de radiothérapie. Les traitements combinés selon la présente invention ont des effets particulièrement efficaces pour la destruction des cellules en hyperprolifération, à des doses relativement faibles. La présente invention fournit ainsi une nouvelle méthode de traitement des pathologies hyperprolifératives (cancers, resténose, etc) particulièrement efficace et avec des effets secondaires limités.

En dépit des progrès très importants réalisés dans ce domaine, les méthodes actuellement disponibles pour le traitement des cancers ont une efficacité toujours limitée. La radiothérapie et la chimiothérapie ont certes un impact très favorable sur le développement des cancers. Cependant, un problème aigü dans le traitement des cancers est l'insensibilité de certaines tumeurs primaires et/ou l'apparition de cellules tumorales résistantes, après un premier cycle de traitements efficaces, à la fois à la radio et à la chimiothérapie.
De nombreuses études ont tenté d'élucider les mécanismes moléculaires qui peuvent être à l'origine de ces évènements. D'une manière générale les travaux ont porté sur la manière dont les agents chimiothérapiques entraient dans les cellules et sur la manière dont ils réagissaient avec leurs cibles cellulaires (Chin et coll., Adv.Cancer Res. 60 (1993) 157-180; Chabner and Meyers in Cancer/ Principles and practices of Oncology, De Vita et al Eds., J.B.Lippencott Co. p. 349-395,1989). Par exemple, de forts niveaux d'expression du gene mdr1 peuvent limiter la concentration intracellulaire d'agents de chimiothérapie divers et pourraient contribuer à l'expression de la résistance multi-drogues. (Chin et coll, voir plus haut ).
Une élucidation plus complète des mécanismes de résistance à la chimiothérapie et à la radiothérapie passe par une meilleure connaissance des processus de mort cellulaire induits par ces agents. Puisque les radiations ionisantes et de nombreux agents anti-cancéreux induisent des domages dans l'ADN, l'effet de ces agents thérapeutiques a été attribué à leur pouvoir génotoxique. Cependant les domages cellulaires causés par ces agents ne permettent pas d'expliquer complètement leur activité thérapeutique (Chabner et Meyers voir plus haut). Ces dernières années, l'exploration et la compréhension des mécanismes de la mort programmée ou apoptose ont permis de reconsidérer les mécanismes par lesquels les cellules tumorales acquièrent ou perdent leur sensibilité aux agents cytotoxiques. De nombreux stimuli toxiques induisent l'apoptose même à des doses insuffisantes pour induire des disfonctionnements métaboliques. La capacité d'induire une réponse apoptotique dans les cellules tumorales pourrait déterminer l'efficacité du traitement.

La demanderesse a maintenant mis au point un nouveau mode de traitement particulièrement efficace pour la destruction des cellules hyperprolifératives. Comme indiqué plus haut, le mode de traitement selon l'invention est basé essentiellement sur l'utilisation combinée de deux types d'agents thérapeutiques : des gènes bloquant des voies de signalisation cellulaires oncogéniques et des agents de chimiothérapie et/ou de radiothérapie. La présente invention découle en effet de la mise en évidence d'un effet synergique particulièrement important lié à l'utilisation combinée de ces deux types d'agents.
Un premier objet de la présente invention concerne donc une association médicamenteuse d'un ou plusieurs acides nucléiques inhibant au moins partiellement des voies de signalisation cellulaires oncogéniques, et d'un agent thérapeutique anticancéreux, pour une utilisation simultanée, séparée ou étalée dans le temps pour le traitement des pathologies hyperprolifératives.
Comme indiqué ci-avant, l'invention repose essentiellement sur la mise en évidence d'un effet synergique entre le produit de certains gènes et les agents de thérapie anticancéreuse. Cette utilisation combinée produit des effets plus puissants, à des doses d'agents inférieures. Cette invention offre donc un moyen pour le traitement des pathologies hyperprolifératives particulièrement avantageux.
Comme indiqué plus loin, selon le gène et l'agent de chimio ou radiothérapie choisis, les deux composantes du traitement combiné de la présente invention peuvent être utilisées de manière simultanée, séparée ou étalée dans le temps. Dans le cas d'une utilisation simultanée, les deux agents sont incubés aux cellules ou administrés au patient simultanément. Selon ce mode de mise en oeuvre de la présente invention, les deux agents peuvent être conditionnés séparément puis mélangés extemporanément avant d'être administrés ensemble. Plus communément, ils sont administrés simultanément mais de manière séparée. En particulier, les vois d'administrations des deux agents peuvent être différentes. Dans un autre mode de mise en oeuvre, les deux agents sont administrés de manière espacée dans le temps.

L'acide nucléique utilisé dans le cadre de la présente invention peut être un acide désoxyribonucléique (ADN) ou un acide ribonucléique (ARN). Parmi les ADN, il peut s'agir d'un ADN complémentaire (ADNc), d'un ADN génomique (ADNg), d'une séquence hybride ou d'une séquence synthétique ou semi-synthétique. Il peut en outre s'agir d'un acide nucléique modifié chimiquement, par exemple en vue d'augmenter sa résistance aux nucléases, sa pénétration ou son ciblage cellulaires, son efficacité thérapeutique, etc. Ces acides nucléiques peuvent être d'origine humaine, animale, végétale, bactérienne, virale, synthétique, etc. Ils peuvent être obtenus par toute technique connue de l'homme du métier, et notamment par criblage de banques, par synthèse chimique, ou encore par des méthodes mixtes incluant la modification chimique ou enzymatiqe de séquences obtenues par criblage de banques. Comme indiqué plus loin, ils peuvent par ailleurs être incorporés dans des vecteurs, tels que des vecteurs plasmidiques, viraux ou chimiques.

Comme indiqué ci-avant, l'acide nucléique selon la présente invention est un acide nucléique capable d'inhiber au moins partiellement des voies de sigalisation cellulaires oncogéniques. Ces acides nucléiques sont désignés dans ce qui suit sous le terme "éléments intracellulaires de neutralisation des oncogènes" ou EINO. Les voies de signalisation conduisant à la transformation cellulaire sont multiples. La prolifération cellulaire met en jeu une multitude de facteurs, tels que des récepteurs membranaires (protéines G), des oncogènes, des enzymes (protéines kinases, farnésyl transférases, phospholipases, etc) des nucléosides (ATP, AMP, GDP, GTP, etc) des facteurs d'activation [facteurs d'échange des guanosines (GRF, GAP, RAF, etc), facteurs transcriptionnels, etc], Des perturbations, par exemple dans la structure, l'activité, la conformation, etc de ces différents facteurs ont été associées à des phénomènes de dérégulation de la prolifération cellulaire. Ainsi, 90 % des adénocarcinomes du pancréas présentent un oncogène Ki-ras muté sur le douzième codon (Almoguera et coll., Cell 53 (1988) 549). De même, la présence d'un gène ras muté a été mise en évidence dans les adénocarcinomes du colon et les cancers de la thyroïde (50 %), ou dans les carcinomes du poumon et les leucémies myéloïdes (30 %, Bos, J.L. Cancer Res. 49 (1989) 4682). De nombreux autres oncogènes ont aujourd'hui été identifiés (myc, fos, jun, ras, myb, erb, etc), dont des formes mutées semblent responsables d'un dérèglement de la prolifération cellulaire. De même, des formes mutées de p53 sont observées dans de nombreux cancers, tels que notamment les cancers colorectaux, les cancers du sein, les cancers du poumon, les cancers gastriques, les cancers de l'oesophage, les lymphômes B, les cancers ovariens, les cancers de la vessie, etc. Les acides nucléiques utilisés dans le cadre de l'invention sont des acides nucléiques capables d'interférer avec l'un de ces facteurs impliqués dans la prolifération cellulaire, et d'inhiber au moins partiellement son activité. Les facteurs préférentiellement visés par les acides nucléiques de l'invention sont ceux qui apparaissent préférentiellement ou spécifiquement lors de dérèglements de la prolifération cellulaire (oncogènes activés, mutant de suppresseur de tumeurs, etc).
Les acides nucléiques utilisés dans le cadre de l'invention peuvent être de différents types. Il s'agit de manière préférentielle :
- d'acides nucléiques antisens,
- d'oligoribonucléotides capables de fixer directement des protéines cibles oncogéniques pour les neutraliser (ARN ligand),
- d'acides nucléiques codant pour des protéines à caractère dominant négatif capables de s'oligomériser, produisant ainsi un complexe inactif,
- d'acides nucléiques codant pour des anticorps intracellulaires (par exemple des fragments variables à chaîne unique issu d'un anticorps) dirigés contre une protéine oncogénique (ScFv).
- de gènes suppresseurs de tumeurs.

Selon un premier mode préféré de mise en oeuvre de la présente invention, l'acide nucléique est un ADN ou un ARN codant pour un polypeptide ou une protéine inhibant au moins partiellement des voies de sigalisation cellulaires oncogéniques. Plus particulièrement, le polypeptide ou la protéine sont choisis parmi les dominants négatifs, les ScFv et les suppresseurs de tumeurs.
Encore plus préférentiellement, le dominant négatif est un constitué de la région N-terminale de la protéine GAP, de la protéine Gbr3-3 ou de mutants des protéines Ets. Concernant le ScFv, il s'agit préférentiellement d'un ScFv dirigé contre une protéine ras mutée ou contre le facteur GAP. La protéine suppresseur de tumeurs est avantageusement p53, Rb, waf1, p21, DCC ou MTS.

Selon un autre mode préféré de mise en oeuvre de la présente invention, l'acide nucléique est un ADN codant pour un ARN inhibant au moins partiellement des voies de signalisation cellulaires oncogéniques. Plus particulièrement, l'ARN est un ARN complémentaire d'un acide nucléique cible et capable de bloquer sa transcription et/ou sa traduction (ARN antisens); un ribozyme ou un ARN ligand. Un exemple préféré est un ARN antisens anti-Kiras.

Toujours selon un mode préféré de mise en oeuvre de la présente invention, l'acide nucléique est un oligonucléotide antisens, éventuellement modifié chimiquement. Il peut s'agir en particulier d'oligonucléotides dont le squelette phosphodiester a été modifié chimiquement, tels que par exemple les oligonucléotides phosphonates, phosphotriesters, phosphoramidates et phosphorothioates qui sont décrits, par exemple, dans la demande de brevet WO94/08003. Il peut également s'agir d'oligonucléotides alpha, ou d'oligonucléotides conjugués à des agents tels que des composés acrylants.

Dans un mode de mise en oeuvre particulièrement préféré de la présente invention l'acide nucléique est incorporé dans un vecteur. Le vecteur utilisé peut être d'origine chimique (liposome, nanoparticule, complexe peptidique, lipides cationiques, etc) virale (rétrovirus, Adénovirus, virus de l'herpès, AAV, virus de la vaccine, etc) ou plasmidique. L'acide nucléique utilisé dans la présente invention peut être formulé en vue d'administrations par voie topique, orale, parentérale, intranasale, intraveineuse, intramusculaire, sous-cutanée, intraoculaire, transdermique, etc. De préférence, l'acide nucléique est utilisé sous une forme injectable. Il peut donc être mélangé à tout véhicule pharmaceutiquement acceptable pour une formulation injectable, notamment pour une injection directe au niveau du site à traiter. Il peut s'agir en particulier de solutions stériles, isotoniques, ou de compositions sèches, notamment lyophilisées, qui, par addition selon le cas d'eau stérilisée ou de sérum physiologique, permettent la constitution de solutés injectables. Une injection directe de l'acide nucléique dans la tumeur du patient est intéressante car elle permet de concentrer l'effet thérapeutique au niveau des tissus affectés. Les doses d'acide nucléique utilisées peuvent être adaptées en fonction de différents paramètres, et notamment en fonction du gène, du vecteur, du mode d'administration utilisé, de la pathologie concernée ou encore de la durée du traitement recherchée.

L'agent thérapeutique anticancéreux utilisé pour la mise en oeuvre de la présente invention peut être tout agent actuellement utilisé par l'homme de l'art dans une chimiothérapie ou une radiothérapie. En particulier, il peut s'agir de cis-platine, taxoïde, étoposide, TNF, adriamycine, camptotécine, d'un poison du fuseau mitotique (vincaalcaloïdes, navelleine, etc), rayons X, UV, etc. Des résultats particulièrement intéressants ont été obtenus en utilisant comme agent chimiothérapeutique un taxoïde. L'Agent chimiothérapeutique anticancéreux est administré selon les voies classiques. Généralement, il est administré par voie parentérale.

Comme indiqué ci-avant, les deux agents peuvent être utilisés de manière simultanée, séparée ou étalée dans le temps. Dans un mode particulièrement préféré de mise en oeuvre de l'invention, l'acide nucléique est administré en premier, puis, lorsque l'acide nucléique peut être exprimé par les ou des cellules, l'agent thérapeutique anticancéreux est administré.

Un mode particulièrement préféré de mise en oeuvre de la présente invention concerne une association médicamenteuse d'un ou plusieurs gènes suppresseurs de tumeurs et d'un taxoïde, pour une utilisation simultannée, séparée ou étalée dans le temps pour le traitement des pathologies hyperprolifératives. Encore plus préférentiellement, le gène suppresseur code pour la forme sauvage de la protéine p53 ou pour la protéine waf1 (p21.)

La présente invention fournit ainsi une méthode particulièrement efficace pour la destruction de cellules hyperprolifératives. Elle peut être utilisée in vitro ou ex vivo, par incubation des cellules de manière simultanée ou étalée dans le temps en présence du ou des acides nucléiques et des agents de chimiothérapie. A cet égard, l'invention a également pour objet une méthode de destruction de cellules hyperprolifératives comprenant la mise en contact desdites cellules ou d'une partie d'entre-elles avec un acide nucléique et un agent de chimiothérapie tels que définis ci-avant.
La présente invention est avantageusement utilisée in vivo pour la destruction de cellules en hyperprolifération (i.e. en prolifération anormale). Elle est ainsi applicable à la destruction des cellules tumorales ou des cellules de muscle lisse de la paroi vasculaire (resténose). Elle est tout particulièrement appropriée au traitement des cancers dans lesquels un oncogène activé est impliqué. A titre d'exemple, on peut citer les adénocarcinomes du colon, les cancers de la thyroïde, les carcinomes du poumon, les leucémies myéloïdes, les cancers colorectaux, les cancers du sein, les cancers du poumon, les cancers gastriques, les cancers de l'oesophage, les lymphômes B, les cancers ovariens, les cancers de la vessie, les glioblastomes, etc.

### Utilisation d'acides nucléiques antisens

La régulation de l'expression de gènes-cible au moyen d'acides nucléiques antisens constitue une approche thérapeutique en développement croissant. Cette approche repose sur la capacité des acides nucléiques à s'hybrider spécifiquement à des régions complémentaires d'un autres acide nucléique, et à inhiber ainsi spécifiquement l'expression de gènes déterminés. Cette inhibition peut intervenir soit au niveau traductionnel soit au niveau transcriptionnel.
Les acides nucléiques antisens sont des séquences nucléiques capables de s'hybrider sélectivement avec des ARNs messager cellulaires-cible, pour inhiber leur traduction en protéine. Ces acides nucléiques forment avec l'ARNm-cible, de manière locale, des régions double-brin, de type ARN/ARNm ou même ADN/ARNm, par interaction de type Watson-Crick classique. Il peut s'agir par exemple d'oligonucléotides synthétiques, de petite taille, complémentaires d'ARNm cellulaires, et qui sont introduits dans les cellules-cible. De tels oligonucléotides ont par exemple été décrits dans le brevet n° EP 92 574. Il peut également s'agir de séquences d'ADN dont l'expression dans la cellule-cible génère des ARN complémentaires d'ARNm cellulaires. De telles séquences ont par exemple été décrites dans le brevet n° EP 140 308.
Plus récemment, un nouveau type d'acides nucléiques capables de réguler l'expression de gènes-cible a été mis en évidence. Ces acides nucléiques ne s'hybrident pas avec les ARNm cellulaires, mais directement avec l'ADN génomique en double-brin. Cette nouvelle approche repose sur la mise en évidence que certains acides nucléiques sont capables d'interagir spécifiquement dans le grand sillon de la double-hélice d'ADN pour former localement des triple-hélices, conduisant à une inhibition de la transcription de gènes-cible. Ces acides nucléiques reconnaissent sélectivement la double-hélice d'ADN au niveau de séquences oligopurine.oligopyrimidine, c'est-à-dire au niveau de régions possédant une séquence oligopurique sur un brin et une séquence oligopyrimidique sur le brin complémentaire, et y forment localement une triple-hélice. Les bases du troisième brin (l'oligonucléotide) forment des liaisons hydrogène (liaisons Hoogsteen ou Hoogsteen inverse) avec les purines des paires de bases Watson-Crick. De tels acides nucléiques ont notamment été décrits par Hélène dans Anti-Cancer drug design 6 (1991) 569.
Les acides nucléiques antisens selon la présente invention peuvent être des séquences d'ADN codant pour des ARN antisens ou pour des ribozymes. Les ARN antisens ainsi produits peuvent interagir avec un ARNm ou un ADN génomique cible et former avec celui-ci des doubles ou des triples hélices. Il peut également s'agir de séquences (oligonucléotides) antisens, éventuellement modifiés chimiquement, capables d'interagir directement avec le gène ou l'ARN cible.
Préférentiellement, les antisens selon l'invention sont dirigés contre des oncogènes ou des régions spécifiques d'oncogènes activés, en particulier l'oncogène ras.

### Utilisation d'ARN ligands

Les ARN ligands sont des oligoribonucléotides de petite taille, très spécifiques et très affins pour une cible donnée, notamment protéique. La préparation et l'identification de tels ARN ligands a été décrite notamment dans la demande WO91/19813. Selon un mode de mise en oeuvre particulier de la présente invention, il est possible d'associer un petit ARN spécifique de la protéine Ki-ras, exprimé dans les cellules par le biais d'un vecteur viral ou non viral adapté, avec les agents de chimiothérapie ou radiothérapie décrits.

### Dominants négatifs

Un dominant négatif est un polypeptideantagoniste d'une voie de signalisation oncogénique. Cet antagonisme intervient lorsque le polypeptide vient se positionner au contact d'un élément clé de la signalisation oncogénique et entre en compétition avec le polypeptide naturellement utilisé dans la cellule pour cette signalisation . Le polypeptide antagoniste utilisé est très fréquemment un mime du polypeptide naturel mais dépourvu des domaines permettant au signal oncogénique de se propager à travers lui .
Parmi les dominants négatifs préférés pour la mise en oeuvre de la présente invention, on peut citer les acides nucléiques codant pour le domaine NH2 terminal de la protéine GAP, pour la protéine Grb3-3 ou pour des formes mutées des protéines ETS.
Il a été démontré dans la demande de brevet WO94/03597 que la surexpression du domaine NH2 terminal de la protéine GAP-Ras pouvait spécifiquement bloquer la tumorigénicité de cellules transformées à la suite de l'expression d'un gène ras muté. L'exemple 1 de la présente demande montre maintenant qu'une surexpression du domaine GAP(170-702) induit une apoptose de cellules humaines, carcinome dit non à petites cellules du poumon (H460). L'exemple 2 montre de plus que l'effet apoptotique induit par la construction GAP (170-702 ) est très largement augmenté par l'ajout dans le milieu de culture des cellules tumorales humaines de produits tels que le cis-platine, la camptotécine ou le taxotère à des concentrations de ces produits qui sont sans effet sur la viabilité cellulaire.
L'exemple 1 de la présente demande décrit par ailleurs l'activité du gène grb3-3 dans les cellules H460. La séquence et la fonction présumée de Grb3-3 ont été decrites dans Science 1994. L'exemple 2 montre également que l'effet apoptotique induit par le transfert du gène Grb3-3 est très largement augmenté par l'ajout dans le milieu de culture des cellules tumorales humaines de produits tels que le cis-platine, la camptotécine ou le taxotère à des concentrations de ces produits qui sont sans effet sur la viabilité cellulaire.

Ces exemples démontrent clairement que différents agents de chimiothérapie peuvent être efficacement associés à des stratégies d'induction d'apoptose par le biais de transfert de gènes.

### ScFv

Les ScFv sont des molécules ayant des propriété de liaison comparables à celle d'un anticorps, actives intracellulairement. Il s'agit plus particulièrement de molécules constituées d'un peptide correspondant au site de liaison de la région variable de la chaine légère d'un anticorps relié par un linker peptidique à un peptide correspondant au site de liaison de la région variable de la chaine lourde d'un anticorps. Il a été montré par la demanderesse que de tels ScFv pouvaient être produits in vivo par transfert de gène (Cf demande WO 94/29446).
Plus particulièrement, cette demande montre qu'il est possible de neutraliser des protéines oncogéniques en exprimant dans différents compartiments cellulaires des ScFv. Selon un mode de mise en oeuvre de la présente invention, on utilise un acide nucléique permettant la production intracellulaire d'un ScFv neutralisant le pouvoir transformant des protéines ras, en combinaison avec un agent de chimiothérapie. Une telle association produit des effets synergiques importants (Cf exemple 2).

### Suppresseurs de tumeurs

Parmi les gènes suppresseur de tumeurs utilisables dans le cadre de la présente invention, on peut citer plus particulièrement les gènes p53, p21, Rb, rap1A, DDC, WAF et MTS. Plus particulièrement, on utilise les gènes p53, Rb ou Waf.
Le gène p53 code pour une protéine nucléaire de 53 kDa. La forme mutée par délétion et/ou mutation de ce gène est impliquée dans le développement de la plupart des cancers humains (Baker et coll., Science 244 (1989) 217). Ses formes mutées sont également capables de coopérer avec les oncogènes ras pour transformer des fibroblastes murins. Le gène sauvage codant pour la p53 native inhibe en revanche la formation des foyers de transformation dans des fibroblastes de rongeurs transfectés avec diverses combinaisons d'oncogènes. Des données récentes soulignent que la protéine p53 pourrait être elle-même un facteur de transcription et stimuler l'expression d'autres gènes suppresseurs de tumeur. Par ailleurs, un effet de p53 sur la prolifération des cellules musculaires lisses vasculaires a été mis en évidence récemment (Epstein et al. Science 151 (1994)).
Le gène Rb détermine la synthèse d'une phosphoprotéine nucléaire de 927 acides aminés environ (Friend et coll., Nature 323 (1986) 643) dont la fonction est de réprimer la division des cellules en les faisant entrer en phase de quiescence. Des formes inactivées du gène Rb ont été mises en cause dans différentes tumeurs, et notamment dans les rétinoblastomes ou dans les cancers mésenchymateux comme les ostéosarcomes. La réintroduction de ce gène dans les cellules tumorales où il était inactivé produit un retour à l'état normal et une perte de la tumorigénicité (Huang et coll., Science 242 (1988) 1563). Récemment, il a été démontré que la protéine Rb normale, mais pas ses formes mutées, réprime l'expression du proto-oncogène c-fos, gène indispensable à la prolifération cellulaire.
Les gènes WAF et MTS et leurs propriétés antitumorales ont été décrits dans la littérature (Cell 75 (1993) 817; Science 264 (1994) 436).

L'exemple 3 démontre un type d'association efficace entre un dérivé du taxol et le gène p53. Le taxol induit l'apoptose dans différentes lignées cellulaires tumorales en culture ( Proceedings of the American Association for cancer Research Vol 35, march 1994,Bhalla et al p306 ,Seiter et al p314, Saunders et al p317). p53 déclenche une apoptose dans différents types cellulaires. Nous avons maintenant pu montrer que l'association d'un dérivé du taxol et de p53 induit une apoptose de cellules tumorales humaines. Notamment, des clones particuliers de cellules H460 résistants à l'effet de p53 ont été cultivés en présence de doses croissantes de taxotère. L'exemple 3 démontre clairement que les cellules meurent à la suite du traitement par le taxotère à des concentrations totalement inefficaces sur les cellules n'exprimant pas de p53 sauvage.
Waf 1 (Wild-type p53 Activated Fragment Cell ,75,817, 1993) ou encore p21 ( Nature ,366,701,1993 ) est induit par la surexpression de p53 sauvage . Waf 1 apparait dans les cellules qui sont arrêtees en phase G1 ou en apoptose à la suite d'une surexpression de p53 sauvage mais pas dans les cellules qui sont arrêtées en G1 ou en apoptose d'une manière indépendante de p 53 ( Cancer Res, 54, 1169, 1994). Waf 1 diminue la croissance des cellules tumorales aussi efficacement que p53 sauvage. L'utilisation combinée d'un gène Waf 1 et de dérivés du taxol induit également un effet synergique sur la destruction des cellules hyperprolifératives.

### Agent thérapeutique anticancéreux

Les agents thérapeutiques anticancéreux utilisables dans la thérapie combinée selon la présente invention peuvent être choisis parmi tous les agents chimiothérapeutique ou radiothérapeutique connus de l'homme du métier. Il peut s'agir notamment de cis-platine, taxoïde, étoposide, TNF, adriamycine, camptotécine, d'un poison du fuseau mitotique, etc. Ces différents agents peuvent être obtenus de source commerciale.
Parmi ces agents, les taxoïdes constituent un mode de réalisation préféré. A cet égard, les taxoïdes qui peuvent plus particulièrement être utilisés dans le cadre de la présente invention sont ceux qui sont représentés par la formule générale : 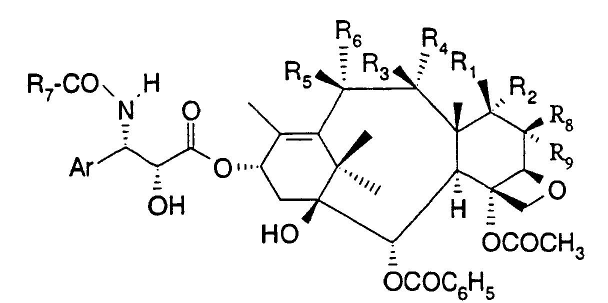 dans laquelle :
- les symboles R₁ et R₂ représentent chacun un atome d'hydrogène, ou bien un des radicaux R₁ ou R₂ représente un atome d'hydrogène, et l'autre représente un radical hydroxy, acyloxy ou acylcarbonyloxy, ou bien R₂ représente un atome d'hydrogène et R₁ forme avec l'atome de carbone du radical méthyle en α une liaison, de façon à former un cycle cyclopropane,
- l'un des symboles R₃ ou R₄ représente un atome d'hydrogène, et l'autre représente un radical hydroxy, ou bien R₃ et R₄ forment ensemble un radical carbonyle,
- les symboles R₅ et R₆ représentent chacun un atome d'hydrogène, ou bien un des symboles R₅ ou R₆ représente un atome d'hydrogène et l'autre représente un radical hydroxy, acyloxy, acylcarbonyloxy ou alcoxyméthylcarbonyloxy, ou bien R₅ et R₆ forment ensemble un radical carbonyle,
- les symboles R₈ et R₉ représentent chacun un atome d'hydrogène ou bien R₁ et R₈ forment ensemble une liaison,
- le symbole R₇ représente un radical alcoxy, alcényloxy ou cycloalcoyloxy ou un radical phényle, et
- Ar représente un radical phényle éventuellement substitué par un ou plusieurs atomes ou radicaux, identiques ou différents, choisis parmi les atomes d'halogène et les radicaux alcoyles, alcoxy, dialcoylamino, acylamino, alcoxycarbonylamino ou trifluorométhyle, ou un radical hétérocyclique aromatique à 5 chaînons contenant un ou plusieurs hétéroatomes, identiques ou différents, choisis parmi les atomes d'azote, d'oxygène et de soufre,
étant entendu que les radicaux alcoyles et les portions alcoyles des autres radicaux contiennent 1 à 8 atomes de carbone en chaîne droite ou ramifiée et que les radicaux alcényles contiennent 2 à 8 atomes de carbone.
Plus particulièrement intéressants sont les taxoïdes pour lesquels R₂ représentant un atome d'hydrogène, R₁ représente un atome d'hydrogène ou un rdical hydroxy ou bien R₁ forme avec l'atome de carbone du radical méthyle en α une simple liaison, R₃ et R₄ forment ensemble un radical carbonyle, R₅ représente un atome d'hydrogène et R₆ représente un atome d'hydrogène ou un radical hydroxy, acétyloxy ou méthoxyacétyloxy ou bien R₅ et R₆ forment ensemble un radical carbonyle, R₇ représente un radical t-butoxy ou un radical phényle.
Peuvent être particulièrement cités les produits suivants :
- t-butoxycarbonylamino-3' phényl-3' hydroxy-2' propionate-(2R,3S) d'acétoxy-4 benzoyloxy-2α époxy-5β,20 trihydroxy-1β,7β,10β oxo-9 taxène-11 yl-13α (docetaxel ou Taxotère®)
- benzoylamino-3' phényl-3' hydroxy-2' propionate-(2R,3S) de diacétoxy-4,10β benzoyloxy-2a époxy-5β,20 dihydroxy-1β,7β oxo-9 taxène-11 yl-13α (paclitaxel)
- t-butoxycarbonylamino-3' phényl-3' hydroxy-2' propionate-(2R,3S) d'acétoxy-4 benzoyloxy-2α époxy-5β,20 dihydroxy-1β,10β méthylène-7β,10β nor-19 oxo-9 taxène-11 yl-13α
- t-butoxycarbonylamino-3' phényl-3' hydroxy-2' propionate-(2R,3S) de diacétoxy-4,10β benzoyloxy-2α époxy-5β,20 hydroxy-1β méthylène-7β,10β nor-19 oxo-9 taxène-11 yl-13α
- t-butoxycarbonylamino-3' (fluoro-2 phényl)-3' hydroxy-2' propionate-(2R,3S) d'acétoxy-4 benzoyloxy-2α époxy-5β,20 trihydroxy-1β,7β,10β oxo-9 taxène-11yl-13α
- t-butoxycarbonylamino-3' (chloro-4 phényl)-3' hydroxy-2' propionate-(2R,3S) d'acétoxy-4 benzoyloxy-2α époxy-5β,20 trihydroxy-1β,7β,10β oxo-9 taxène-11 yl-13α
- t-butoxycarbonylamino-3' (méthoxy-4 phényl)-3' hydroxy-2' propionate-(2R,3S) d'acétoxy-4 benzoyloxy-2α époxy-5β,20 trihydroxy-1β,7β,10β oxo-9 taxène-11 yl-13α
- t-butoxycarbonylamino-3' (fluoro-4 phényl)-3' hydroxy-2' propionate-(2R,3S) d'acétoxy-4 benzoyloxy-2α époxy-5β,20 trihydroxy-1β,7β,10β oxo-9 taxène-11 yl-13α
- adamantyloxycarbonylamino-3' phényl-3' hydroxy-2' propionate-(2R,3S) d'acétoxy-4 benzoyloxy-2α époxy-5β,20 trihydroxy-1β,7β,10β oxo-9 taxène-11 yl-13α
- tert-pentyloxycarbonylamino-3' phényl-3' hydroxy-2' propionate-(2R,3S) d'acétoxy-4 benzoyloxy-2α époxy-5β,20 trihydroxy-1β,7β,10β oxo-9 taxène-11 yl-13α
- (méthyl-1 cyclohexyl)oxycarbonylamino-3' phényl-3' hydroxy-2' propionate-(2R,3S) d'acétoxy-4 benzoyloxy-2α époxy-5β,20 trihydroxy-1β,7β,10β oxo-9 taxène-11 yl-13α
- (méthyl-1 cyclopropyl)oxycarbonylamino-3' phényl-3' hydroxy-2' propionate-(2R,3S) d'acétoxy-4 benzoyloxy-2α époxy-5β,20 trihydroxy-1β,7β,10β oxo-9 taxène-11 yl-13α
- (méthyl-1 cyclopentyl)oxycarbonylamino-3' phényl-3' hydroxy-2' propionate-(2R,3S) d'acétoxy-4 benzoyloxy-2α époxy-5β,20 trihydroxy-1β,7β,10β oxo-9 taxène-11 yl-13α
- (diméthyl-1,1 propyne-2)yloxycarbonylamino-3' phényl-3' hydroxy-2' propionate-(2R,3S) d'acétoxy-4 benzoyloxy-2α époxy-5β,20 trihydroxy-1β,7β,10β oxo-9 taxène-11 yl-13α
- t-butoxycarbonylamino-3' phényl-3' hydroxy-2' propionate-(2R,3S) d'acétoxy-4 benzoyloxy-2α époxy-5β,20 tétrahydroxy-1β,7β,9β,10β taxène-11 yl-13α
- t-butoxycarbonylamino-3' phényl-3' hydroxy-2' propionate-(2R,3S) d'acétoxy-4 benzoyloxy-2α époxy-5β,20 dihydroxy-1β,7β oxo-9 taxène-11 yl-13α
- t-butoxycarbonylamino-3' (thiényl-2)-3' hydroxy-2' propionate-(2R,3S) d'acétoxy-4 benzoyloxy-2α époxy-5β,20 trihydroxy-1β,7β,10β oxo-9 taxène-11 yl-13α
- t-butoxycarbonylamino-3' (furyl-2)-3' hydroxy-2' propionate-(2R,3S) d'acétoxy-4 benzoyloxy-2α époxy-5β,20 trihydroxy-1β,7β,10β oxo-9 taxène-11 yl-13α
- t-butoxycarbonylamino-3' (thiényl-3)-3' hydroxy-2' propionate-(2R,3S) d'acétoxy-4 benzoyloxy-2α époxy-5β,20 trihydroxy-1β,7β,10β oxo-9 taxène-11 yl-13α
- t-butoxycarbonylamino-3' phényl-3' hydroxy-2' propionate-(2R,3S) d'acétoxy-4 benzoyloxy-2α époxy-5β,20 dihydroxy-1β,10β oxo-9 taxène-11 yl-13α
- t-butoxycarbonylamino-3' phényl-3' hydroxy-2' propionate-(2R,3S) d'acétoxy-4 benzoyloxy-2α époxy-5β,20 dihydroxy-1β,7β dioxo-9,10 taxène-11 yl-13α
- t-butoxycarbonylamino-3' phényl-3' hydroxy-2' propionate-(2R,3S) d'acétoxy-4 benzoyloxy-2α époxy-5β,20 hydroxy-1β oxo-9 taxène-11 yl-13α

Ces différents composés peuvent être obtenus selon les méthodes décrites dans les demandes WO94/13654 et WO92/09589 par exemple.

Il est particulièrement avantageux au sens de la présente invention d'utiliser le taxol, le docetaxel ou le paclitaxel.

### Vecteurs d'administration de l'acide nucléique

L'acide nucléique peut être injecté tel quel au niveau du site à traiter, ou incubé directement avec les cellules à détruire ou traiter. Il a en effet été décrit que les acides nucléiques nus pouvaient pénétrer dans les cellules sans vecteur particulier. Néanmoins, on préfère dans le cadre de la présente invention utiliser un vecteur d'administration, permettant d'améliorer (i) l'efficacité de la pénétration cellulaire, (ii) le ciblage (iii) la stabilité extra- et intracellulaires.
Différents types de vecteurs peuvent être utilisés. Il peut s'agir de vecteurs viraux ou non viraux.

### Vecteurs viraux

L'utilisation de vecteurs viraux repose sur les propriétés naturelles de transfection des virus. Il est ainsi possible d'utiliser les adénovirus, les herpès virus, les rétrovirus et plus récemment les virus adéno associés. Ces vecteurs s'avèrent particulièrement performants sur le plan de la transfection.

S'agissant plus particulièrement d'adénovirus, différents sérotypes, dont la structure et les propriétés varient quelque peu, ont été caractérisés. Parmi ces sérotypes, on préfère utiliser dans le cadre de la présente invention les adénovirus humains de type 2 ou 5 (Ad 2 ou Ad 5) ou les adénovirus d'origine animale (voir demande WO94/26914). Parmi les adénovirus d'origine animale utilisables dans le cadre de la présente invention on peut citer les adénovirus d'origine canine, bovine, murine, (exemple : Mav1, Beard et al., Virology 75 (1990) 81), ovine, porcine, aviaire ou encore simienne (exemple : SAV). De préférence, l'adénovirus d'origine animale est un adénovirus canin, plus préférentiellement un adénovirus CAV2 [souche manhattan ou A26/61 (ATCC VR-800) par exemple]. De préférence, on utilise dans le cadre de l'invention des adénovirus d'origine humaine ou canine ou mixte.

Préférentiellement, les adénovirus défectifs de l'invention comprenent les ITR, une séquence permettant l'encapsidation et l'acide nucléique d'intérêt. Encore plus préférentiellement, dans le génome des adénovirus de l'invention, la région E1 au moins est non fonctionnelle. Le gène viral considéré peut être rendu non fonctionnel par toute technique connue de l'homme du métier, et notamment par suppression totale, substitution, délétion partielle, ou addition d'une ou plusieurs bases dans le ou les gènes considérés. De telles modifications peuvent être obtenues in vitro (sur de l'ADN isolé) ou in situ, par exemple, au moyens des techniques du génie génétique, ou encore par traitement au moyen d'agents mutagènes. D'autres régions peuvent également être modifiées (E2, E3, E4, L1-L5, etc).
Les adénovirus recombinants défectifs selon l'invention peuvent être préparés par toute technique connue de l'homme du métier (Levrero et al., Gene 101 (1991) 195, EP 185 573; Graham, EMBO J. 3 (1984) 2917). En particulier, ils peuvent être préparés par recombinaison homologue entre un adénovirus et un plasmide portant entre autre la séquence d'ADN d'intérêt. La recombinaison homologue se produit après co-transfection desdits adénovirus et plasmide dans une lignée cellulaire appropriée. La lignée cellulaire utilisée doit de préférence (i) être transformable par lesdits éléments, et (ii), comporter les séquences capables de complémenter la partie du génome de l'adénovirus défectif, de préférence sous forme intégrée pour éviter les risques de recombinaison. A titre d'exemple de lignée, on peut mentionner la lignée de rein embryonnaire humain 293 (Graham et al., J. Gen. Virol. 36 (1977) 59) qui contient notamment, intégrée dans son génome, la partie gauche du génome d'un adénovirus Ad5 (12 %). Des stratégies de construction de vecteurs dérivés des adénovirus ont également été décrites dans les demandes n° WO 94/26914 et FR 93 08596.
Ensuite, les adénovirus qui se sont multipliés sont récupérés et purifiés selon les techniques classiques de biologie moléculaire, comme illustré dans les exemples.

Concernant les virus adéno-associés (AAV), il s'agit de virus à ADN de taille relativement réduite, qui s'intègrent dans le génome des cellules qu'ils infectent, de manière stable et site-spécifique. Ils sont capables d'infecter un large spectre de cellules, sans induire d'effet sur la croissance, la morphologie ou la différenciation cellulaires. Par ailleurs, ils ne semblent pas impliqués dans des pathologies chez l'homme. Le génome des AAV a été cloné, séquencé et caractérisé. Il comprend environ 4700 bases, et contient à chaque extrémité une région répétée inversée (ITR) de 145 bases environ, servant d'origine de réplication pour le virus. Le reste du génome est divisé en 2 régions essentielles portant les fonctions d'encapsidation : la partie gauche du génome, qui contient le gène rep impliqué dans la réplication virale et l'expression des gènes viraux; la partie droite du génome, qui contient le gène cap codant pour les protéines de capside du virus.

L'utilisation de vecteurs dérivés des AAV pour le transfert de gènes in vitro et in vivo a été décrite dans la littérature (voir notamment WO 91/18088; WO 93/09239; US 4,797,368, US5,139,941, EP 488 528). Ces demandes décrivent différentes constructions dérivées des AAV, dans lesquelles les gènes rep et/ou cap sont délétés et remplacés par un gène d'intérêt, et leur utilisation pour transférer in vitro (sur cellules en culture) ou in vivo (directement dans un organisme) ledit gène d'intérêt. Les AAV recombinants défectifs selon l'invention peuvent être préparés par co-transfection, dans un lignée cellulaire infectée par un virus auxiliaire humain (par exemple un adénovirus), d'un plasmide contenant la séquence nucléique d'intérêt bordée de deux régions répétées inversées (ITR) d'AAV, et d'un plasmide portant les gènes d'encapsidation (gènes rep et cap) d'AAV. Les AAV recombinants produits sont ensuite purifiés par des techniques classiques.
Concernant les virus de l'herpès et les rétrovirus, la construction de vecteurs recombinants a été largement décrite dans la littérature : voir notamment Breakfield et al., New Biologist 3 (1991) 203; EP 453242, EP178220, Bernstein et al. Genet. Eng. 7 (1985) 235; McCormick, BioTechnology 3 (1985) 689, etc. En particulier, les rétrovirus sont des virus intégratifs, infectant sélectivement les cellules en division. Ils constituent donc des vecteurs d'intérêt pour des applications cancer. Le génome des rétrovirus comprend essentiellement deux LTR, une séquence d'encapsidation et trois régions codantes (gag, pol et env). Dans les vecteurs recombinants dérivés des rétrovirus, les gènes gag, pol et env sont généralement délétés, en tout ou en partie, et remplacés par une séquence d'acide nucléique hétérologue d'intérêt. Ces vecteurs peuvent être réalisés à partir de différents types de rétrovirus tels que notamment le MoMuLV ("murine moloney leukemia virus"; encore désigné MoMLV), le MSV ("murine moloney sarcoma virus"), le HaSV ("harvey sarcoma virus"); le SNV ("spleen necrosis virus"); le RSV ("rous sarcoma virus") ou encore le virus de Friend.
Pour construire des rétrovirus recombinants comportant une séquence d'intérêt, un plasmide comportant notamment les LTR, la séquence d'encapsidation et ladite séquence d'intérêt est généralement construit, puis utilisé pour transfecter une lignée cellulaire dite d'encapsidation, capable d'apporter en trans les fonctions rétrovirales déficientes dans le plasmide. Généralement, les lignées d'encapsidation sont donc capables d'exprimer les gènes gag, pol et env. De telles lignées d'encapsidation ont été décrites dans l'art antérieur, et notamment la lignée PA317 (US4,861,719); la lignée PsiCRIP (WO90/02806) et la lignée GP+envAm-12 (WO89/07150). Par ailleurs, les rétrovirus recombinants peuvent comporter des modifications au niveau des LTR pour supprimer l'activité transcriptionnelle, ainsi que des séquences d'encapsidation étendues, comportant une partie du gène gag (Bender et al., J. Virol. 61 (1987) 1639). Les rétrovirus recombinants produits sont ensuite purifiés par des techniques classiques.

Pour la mise en oeuvre de la présente invention, il est tout particulièrement avantageux d'utiliser un adénovirus ou un rétrovirus recombinant défectif. Ces vecteurs possèdent en effet des propriétés particulièrement intéressantes pour le transfert de gènes dans les cellules tumorales.

### Vecteurs non-viraux

Le vecteur selon l'invention peut également être un agent non viral capable de promouvoir le transfert et l'expression d'acides nucléiques dans des cellules eucaryotes. Les vecteurs chimiques ou biochimiques représentent une alternative intéressante aux virus naturels en particulier pour des raisons de commodité, de sécurité et également par l'absence de limite théorique en ce qui concerne la taille de l'ADN à transfecter.
Ces vecteurs synthétiques ont deux fonctions principales, compacter l'acide nucléique à transfecter et promouvoir sa fixation cellulaire ainsi que son passage à travers la membrane plasmique et, le cas échéant, les deux membranes nucléaires. Pour pallier à la nature polyanionique des acides nucléiques, les vecteurs non viraux possèdent tous des charges polycationiques.

Parmi les vecteurs synthétiques développés, les polymères cationiques de type polylysine, (LKLK)n, (LKKL)n, polyéthylène immine et DEAE dextran ou encore les lipides cationiques ou lipofectants sont les plus avantageux. Ils possèdent la propriété de condenser l'ADN et de promouvoir son association avec la membrane cellulaire. Parmi ces derniers, on peut citer les lipopolyamines (lipofectamine, transfectam, etc) et différents lipides cationiques ou neutres (DOTMA, DOGS, DOPE, etc). Plus récemment, il a été développé le concept de la transfection ciblée, médiée par un récepteur, qui met à profit le principe de condenser l'ADN grâce au polymère cationique tout en dirigeant la fixation du complexe à la membrane grâce à un couplage chimique entre le polymère cationique et le ligand d'un récepteur membranaire, présent à la surface du type cellulaire que l'on veut greffer. Le ciblage du récepteur à la transferrine, à l'insuline ou du récepteur des asialoglycoprotéines des hépatocytes a ainsi été décrit.

### Protocole d'administration

Un protocole d'administration préféré selon l'invention comprend d'abord l'acide nucléique, ensuite l'agent thérapeutique. Dans une utilisation préférentielle l'administration du transgène est répétée afin d'obtenir une expression maximum dans un maximum de cellules en division ( par exemple 5 jours de suite ) et le traitement par chimiothérapie est ensuite administré .
Avantageusement, le ou les acides nucléiques sont administrés au contact de la lésion, soit par injection intra-tumorale directe dans des sites multiples de la lésion , soit au contact de la lésion athéromateuse par le moyen d'un coussinet adapté à ce type d'intervention . L'agent de chimiothérapie est administré selon les protocoles cliniques en vigueur .

### Exemples

### Exemple 1

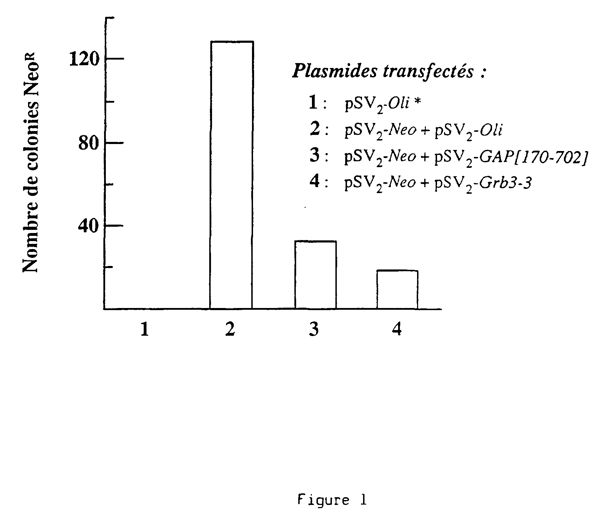

Les cellules H460, cultivées en milieu RPMI 1640 contenant 10% de sérum de veau foetal, sont transfectées avec les cDNAs codant pour le domaine GAP[170-702] ou pour la protéine Grb3-3 en association avec un gène conférant aux cellules positivement transfectées une résistance à la généticine (*Neo*). Ces cDNAs, placés dans des plasmides et dont l'expression est sous le contrôle de promoteurs viraux (pSV₂-*GAP[170-702],* pSV2-*Grb3-3* et pSV2*-Neo*), sont introduits dans les cellules H460 à l'aide de la lipofectAMINE comme agent transfectant. Les cellules H460/Neo^{R} (résistantes à la présence de 400 µg/ml de généticine dans le milieu de culture) sont sélectionnées et quantifiées 15-20 jours après transfection. Les résultats d'une expérience représentative de quantification du nombre de colonies Neo^{R} dans les différentes conditions de transfection sont résumés dans la figure 1 (pSV₂-Oli : plasmide contrôle ne possédant aucun cDNA d'intéret et permettant ainsi le contrôle de l'efficacité de la sélection par la généticine).

### Exemple 2

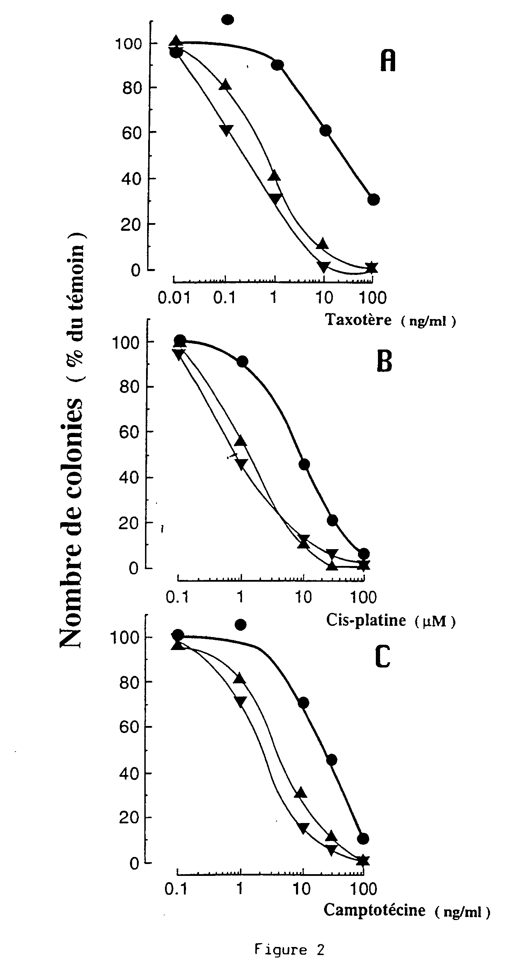

Les cellules H460, transfectées comme indiqué dans l'exemple 1, sont soumises au cours de la sélection par la génécitine à un traitement pendant plusieurs jours à différentes concentrations de taxotère, de cis-platine ou de camptotécine. Les cellules H460/Neo^{R} et résistantes aux agents de chimiothérapie sont quantifiées comme il est décrit dans l'exemple 1. La sensibilité au taxotère (**A**), au cis-platine (**B**) ou à la camptotécine (**C**) des cellules H460 transfectées avec pSV₂-*Neo* (●), pSV2-*Neo* + pSV2-*GAP[170-702]* (▲), ou pSV2-*Neo* + pSV2-*Grb3-3* (▼) est représentée relativement aux cellules transfectées de manière identique mais en absence de traitement aux agents chimiothérapeutiques. Les résultats d'une expérience représentative de quantification du nombre de colonie après les différents traitements indiqués sont résumés dans la figure 2.

### Exemple 3

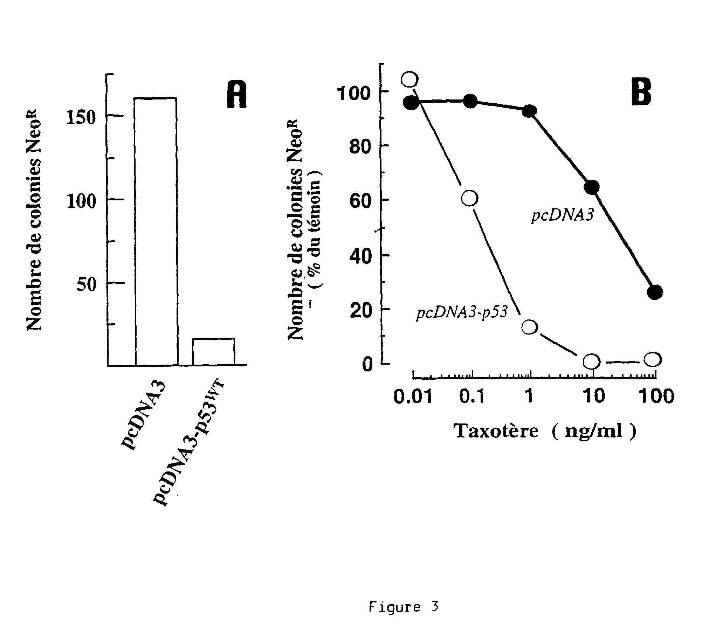

Les cellules H460 sont transfectées avec le cDNA codant pour la protéine p53 sauvage (p53^{WT}) placé dans le plasmide pcDNA3 sous le contôle du promoteur CMV. Le plasmide pcDNA3 contient aussi le gène *Neo* placé sous le contrôle du promoteur SV₄₀. Les cellules transfectées par pcDNA3 ou pcDNA3-p53^{WT} sont sélectionnées et isolées comme il est indiqué dans l'exemple 1. Dans les cellules transfectées par pcDNA3-p53^{WT} et résistantes à la généticine, le présence de p53 est vérifiée par Western blot à l'aide d'anticorps spécifiques. La figure 3 résume les résultats d'une expérience représentative dans laquelle sont représentés, d'une part, le nombre de colonies obtenues après transfection de pcDNA3 ou pcDNA3-p53^{WT} (**A**) et, d'autre part, la sensibilité des clones isolés à un traitement au taxotère (**B**).

## Revendications

1. Association médicamenteuse d'un ou plusieurs acides nucléiques inhibant au moins partiellement les voies de signalisation cellulaires oncogéniques, et d'un agent thérapeutique anticancéreux, pour une utilisation simultanée, séparée ou étalée dans le temps pour le traitement des pathologies hyperprolifératives.

2. Association selon la revendication 1 **caractérisée en ce que** l'acide nucléique est un acide désoxyribonucléique (ADN) ou ribonucléique (ARN) codant pour un produit inhibant au moins partiellement les voies de signalisation cellulaires oncogéniques.

3. Association selon la revendication 2 **caractérisée en ce que** l'acide nucléique est un ADN codant pour un ARN antisens.

4. Association selon la revendication 2 **caractérisée en ce que** l'acide nucléique est un ADN codant pour un ARN ligand.

5. Association selon la revendication 2 **caractérisée en ce que** l'acide nucléique est un ADN codant pour un dominant négatif.

6. Association selon la revendication 2 **caractérisée en ce que** l'acide nucléique est un ADN codant pour un ScFv.

7. Association selon la revendication 2 **caractérisée en ce que** l'acide nucléique est un ADN codant pour une protéine suppresseur de tumeurs.

8. Association selon la revendication 1 **caractérisée en ce que** l'acide nucléique est un oligonucléotide antisens, éventuellement modifié chimiquement.

9. Association selon l'une des revendications précédentes **caractérisée en ce que** l'acide nucléique est incorporé dans un vecteur.

10. Association selon la revendication 9 **caractérisée en ce que** le vecteur est choisi parmi les liposome, nanoparticule, complexe peptidique, lipides cationiques et lipopolyamines.

11. Association selon la revendication 9 **caractérisée en ce que** le vecteur est un vecteur viral dérivé des rétrovirus, adénovirus, virus de l'herpès, AAV, virus de la vaccine.

12. Association selon l'une des revendications précédentes **caractérisée en ce que** l'acide nucléique est administré directement au niveau du site à traiter.

13. Association selon la revendication 1 **caractérisée en ce que** l'agent thérapeutique anticancéreux est un agent chimiothérapeutique choisi parmi le cis-platine, les taxoïdes, l'étoposide, le TNF, l'adriamycine, la camptotécine, les vinca alcaloïdes et la navelleine.

14. Association selon la revendication 13 **caractérisée en ce que** l'agent chimiothérapeutique anticancéreux est un taxoïde.

15. Association selon la revendication 14 **caractérisée en ce que** l'agent chimiothérapeutique anticancéreux est choisi parmi le taxol, le docetaxel et le paclitaxel.

16. Association selon l'une des revendications 13 à 15 **caractérisée en ce que** l'agent chimiothérapeutique anticancéreux est administré par voie parentérale.

17. Association selon l'une des revendications précédentes **caractérisée en ce que** l'acide nucléique et l'agent chimiothérapeutique anticancéreux sont utilisés simultanément.

18. Association selon l'une des revendications 1 à 16 **caractérisée en ce que** l'acide nucléique est administré avant l'agent chimiothérapeutique anticancéreux.

19. Association médicamenteuse d'un ou plusieurs gènes suppresseurs de tumeurs et d'un taxoïde, pour une utilisation simultannée, séparée ou étalée dans le temps pour le traitement des pathologies hyperprolifératives.

20. Association selon revendication 19 **caractérisée en ce que** le gène suppresseur code pour la forme sauvage de la protéine p53.

21. Association selon revendication 19 **caractérisée en ce que** le gène suppresseur code pour la protéine waf1.

22. Association entre la revendication 1 **caractérisé en ce que** l'agent thérapeutique anticancéreux est un agent de radiothérapie.

## Claims

1. Medicinal combination of one or more nucleic acids that at least partially inhibit the oncogenic cell signalling pathways and an anticancer therapeutic agent, for use simultaneously, separately or spread over time for the treatment of hyperproliferative pathologies.

2. Combination according to Claim 1, **characterized in that** the nucleic acid is a deoxyribonucleic acid (DNA) or ribonucleic acid (RNA) coding for a product that at least partially inhibits the oncogenic cell signalling pathways.

3. Combination according to Claim 2, **characterized in that** the nucleic acid is a DNA coding for an antisense RNA.

4. Combination according to Claim 2, **characterized in that** the nucleic acid is a DNA coding for a ligand RNA.

5. Combination according to Claim 2, **characterized in that** the nucleic acid is a DNA coding for a dominant negative.

6. Combination according to Claim 2, **characterized in that** the nucleic acid is a DNA coding for an ScFv.

7. Combination according to Claim 2, **characterized in that** the nucleic acid is a DNA coding for a tumour suppressor protein.

8. Combination according to Claim 1, **characterized in that** the nucleic acid is an antisense oligonucleotide, where appropriate chemically modified.

9. Combination according to one of the preceding claims, **characterized in that** the nucleic acid is incorporated in a vector.

10. Combination according to Claim 9, **characterized in that** the vector is chosen from liposome, nanoparticle, peptide complex, cationic lipids and lipopolyamines.

11. Combination according to Claim 9, **characterized in that** the vector is a viral vector derived from retroviruses, adenovirus, herpesvirus, AAV or vaccinia virus.

12. Combination according to one of the preceding claims, **characterized in that** the nucleic acid is administered directly at the site to be treated.

13. Combination according to Claim 1, **characterized in that** the anticancer therapeutic agent is a chemotherapeutic agent chosen from cisplatin, taxoids, etoposide, TNF, adriamycin, camptothecin, vinca alkaloids and navellein.

14. Combination according to Claim 13, **characterized in that** the anticancer chemotherapeutic agent is a taxoid.

15. Combination according to Claim 14, **characterized in that** the anticancer chemotherapeutic agent is chosen from taxol, docetaxel and paclitaxel.

16. Combination according to one of Claims 13 to 15, **characterized in that** the anticancer chemotherapeutic agent is administered parenterally.

17. Combination according to one of the preceding claims, **characterized in that** the nucleic acid and the anticancer chemotherapeutic agent are used simultaneously.

18. Combination according to one of Claims 1 to 16, **characterized in that** the nucleic acid is administered before the anticancer chemotherapeutic agent.

19. Medicinal combination of one or more tumour suppressor genes and a taxoid, for use simultaneously, separately or spread over time for the treatment of hyperproliferative pathologies.

20. Combination according to Claim 19, **characterized in that** the suppressor gene codes for the wild-type form of the p53 protein.

21. Combination according to Claim 19, **characterized in that** the suppressor gene codes for the waft protein.

22. Combination according to Claim 1, **characterized in that** the anticancer therapeutic agent is a radiotherapeutic agent.

## Patentansprüche

1. Arzneimittel-Assoziation von einer oder mehreren Nucleinsäuren, die mindestens teilweise die Wege der onkogenischen zellularen Signalisierung inhibieren, und einem antikanzerogenen therapeutischen Mittel für eine gleichzeitige, getrennte oder zeitlich ausgedehnte Anwendung zur Behandlung von hyperproliferativen Erkrankungen.

2. Assoziation nach Anspruch 1, **dadurch gekennzeichnet, daß** die Nucleinsäure eine Desoxyribonucleinsäure (DNA) oder eine Ribonucleinsäure (RNA) ist, die für ein Produkt kodieren, das mindestens teilweise die Wege der onkogenischen zellularen Signalisierung inhibiert.

3. Assoziation nach Anspruch 2, **dadurch gekennzeichnet, daß** die Nucleinsäure eine DNA ist, die für eine Gegensinn-RNA kodiert.

4. Assoziation nach Anspruch 2, **dadurch gekennzeichnet, daß** die Nucleinsäure eine DNA ist, die für eine Liganden-RNA kodiert.

5. Assoziation nach Anspruch 2, **dadurch gekennzeichnet, daß** die Nucleinsäure eine DNA ist, die für einen negativen Dominanten kodiert.

6. Assoziation nach Anspruch 2, **dadurch gekennzeichnet, daß** die Nucleinsäure eine DNA ist, die für ein ScFv kodiert.

7. Assoziation nach Anspruch 2, **dadurch gekennzeichnet, daß** die Nucleinsäure eine DNA ist, die für ein Suppressor-Protein von Tumoren kodiert.

8. Assoziation nach Anspruch 1, **dadurch gekennzeichnet, daß** die Nucleinsäure ein Gegensinn-Oligonucleotid ist, gegebenenfalls chemisch modifiziert.

9. Assoziation nach irgendeinem der vorstehenden Ansprüche, **dadurch gekennzeichnet, daß** die Nucleinsäure in einen Vektor inkorporiert wird.

10. Assoziation nach Anspruch 9, **dadurch gekennzeichnet, daß** der Vektor unter den Liposomen, den Nanopartikeln, den Peptidkomplexen, den kationischen Lipiden und den Lipopolyaminen ausgewählt wird.

11. Assoziation nach Anspruch 9, **dadurch gekennzeichnet, daß** der Vektor ein viraler Vektor ist, abgeleitet von Retrovirus, Adenovirus, Herpesvirus, AAV, Kuhpockenvirus.

12. Assoziation nach irgendeinem der vorstehenden Ansprüche, **dadurch gekennzeichnet, daß** die Nucleinsäure direkt in den Bereich der zu behandelnden Stelle verabreicht wird.

13. Assoziation nach Anspruch 1, **dadurch gekennzeichnet, daß** das antikanzerogene therapeutische Mittel ein chemotherapeutisches Mittel ist, ausgewählt unter cis-Platin, den Taxoiden, Etoposid, TNF, Adriamycin, Camptothecin, den Vinca-Alkaloiden und Navellein.

14. Assoziation nach Anspruch 13, **dadurch gekennzeichnet, daß** das antikanzerogene chemotherapeutische Mittel ein Taxoid ist.

15. Assoziation nach Anspruch 14, **dadurch gekennzeichnet, daß** das antikanzerogene chemotherapeutische Mittel unter Taxol, Docetaxel und Paclitaxel ausgewählt wird.

16. Assoziation nach einem der Ansprüche 13 bis 15, **dadurch gekennzeichnet, daß** das antikanzerogene chemotherapeutische Mittel auf parenteralem Weg verabreicht wird.

17. Assoziation nach einem der vorstehenden Ansprüche, **dadurch gekennzeichnet, daß** die Nucleinsäure und das antikanzerogene chemotherapeutische Mittel gleichzeitig angewendet werden.

18. Assoziation nach einem der Ansprüche 1 bis 16, **dadurch gekennzeichnet, daß** die Nucleinsäure vor dem antikanzerogenen chemotherapeutischen Mittel verabreicht wird.

19. Arzneimittel-Assoziation von einem oder mehreren Suppressor-Genen von Tumoren und einem Taxoid für eine gleichzeitige, getrennte oder zeitlich ausgedehnte Anwendung zur Behandlung von hyperproliferativen Erkrankungen.

20. Assoziation nach Anspruch 19, **dadurch gekennzeichnet, daß** das Suppressor-Gen für die wilde Form des Proteins p53 kodiert.

21. Assoziation nach Anspruch 19, **dadurch gekennzeichnet, daß** das Suppressor-Gen für das Protein waf1 kodiert.

22. Assoziation nach Anspruch 1, **dadurch gekennzeichnet, daß** das antikanzerogene therapeutische Mittel ein Mittel der Strahlentherapie ist.
